# DEMANDE DE BREVET EUROPEEN

(11) **EP 1 669 054 A2**
(43) Date de publication de la demande: **14.06.2006**
(21) Numéro de dépôt: 05300983.3
(22) Date de dépôt: 30.11.2005
(51) Int. Cl.: A61K 8/11

(54) **Composition cosmétique comprenant des capsules renfermant un ou plusieurs actifs, et utilisation**

(30) Priorité: 30.11.2004 FR 0452822
(71) Demandeur: L'OREAL, 75008 Paris (FR)
(72) Inventeur: Mathonneau, Estelle, 75010, PARIS (FR); Rollat-Corvol, Isabelle, 75017, PARIS (FR)
(74) Mandataire: Catherine, Alain

(57) **Abrégé**

L'invention concerne une composition cosmétique comprenant un milieu cosmétiquement acceptable, et :
a) au moins un polymère fixant et/ou au moins un agent épaississant,
b) un ou plusieurs types de capsules, chaque capsule comprenant une enveloppe imperméable constituée d'un ou plusieurs polymères réticulés, lesdites capsules contenant un milieu liquide contenant au moins un actif liquide ou constitué d'au moins un actif, le ou les actifs contenus dans un type de cellules étant différents du ou des actifs contenus dans un autre type de capsules, lesdites capsules contenant à titre d'actif(s) :
   b1) au moins un polymère fixant, ou
   b2) au moins un polymère épaississant, ou
   b3) au moins un mono ou polyalcool, ou
   b4) au moins un premier polymère réactif et au moins un deuxième polymère réactif pouvant réagir avec le ou lesdits premiers polymères réactifs, le ou lesdits premiers polymères réactifs étant isolés du ou des deuxièmes polymères réactifs,
lesdites capsules étant capables de libérer le ou les actifs qu'elle contiennent par rupture au moins partielle de l'enveloppe, la rupture de l'enveloppe pour chaque capsule pouvant s'effectuer aussi bien par l'action intentionnelle d'un utilisateur que sans action intentionnelle d'un utilisateur.

## Description

La présente invention concerne une composition cosmétique comprenant des capsules renfermant un ou plusieurs actifs cosmétiques particuliers, ainsi que l'utilisation de ladite composition pour la mise en forme ou le traitement des cheveux.

Les produits de coiffage permettent une mise en forme non permanente des cheveux. Ils s'utilisent sur des cheveux mouillés ou secs avant la mise en forme à la main ou à l'aide d'une brosse ou d'un peigne. Ils contiennent un ou plusieurs actifs cosmétiques tels que des polymères fixants, des épaississants, du glycérol, des silicones ou des cires par exemple.

Après leur application sur les cheveux et après séchage, ces produits durcissent de façon importante. Ils peuvent alors perdre dans le temps leurs effets, fixant, humidifiant, collant, par exemple.

Les produits de permanente permettent une mise en forme durable de la chevelure.

Généralement, la technique utilisée pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres moyens de mise sous tension) une composition réductrice (étape de réduction) puis, de préférence après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée.

La nouvelle forme imposée aux cheveux par un traitement chimique tel que ci-dessus est durable dans le temps et résiste notamment à l'action des lavages à l'eau ou par shampooings.

Cependant, une telle technique ne donne pas entièrement satisfaction. En effet, cette technique est très efficace pour modifier la forme des cheveux, mais très dégradante pour les fibres capillaires.

Il existe donc un besoin d'améliorer la persistance de l'efficacité des produits de coiffage dans le temps, notamment en terme d'effet coiffant et d'effet humidifiant.

On connaît par ailleurs du document US 2003/0195133 des compositions comprenant des nanosphères solides comprenant un parfum, encapsulées dans des microsphères. Les nanosphères solides sont libérées des microsphères par variation du pH ou de la concentration en sel du milieu. Ces compositions permettent la libération contrôlée dans le temps de parfum dans du linge, dans les cheveux ou sur la peau.

On connaît également du document WO 01/74311 des compositions pour augmenter le volume des cheveux, comprenant des microparticules sphériques, un polymère soluble dans l'eau ou gonflable dans l'eau, et un véhicule aqueux, la combinaison du polymère et des particules résultant en un réseau sous forme de film. Les compositions peuvent encore comprendre un fluide encapsulé dans des microcapsules. L'enveloppe des microcapsules est constituée d'un matériau thermoplastique, et est flexible, c'est-à-dire que les microcapsules peuvent être aisément comprimées, et retrouvent leur volume initial lorsque la pression disparaît.

On connaît enfin du document US 3,729,569 une composition cosmétique pour le démaquillage des ongles, comprenant des capsules contenant un solvant et des capsules contenant un parfum.

Cependant, aucun de ces documents ne décrit de compositions permettant de résoudre de manière satisfaisante les problèmes évoqués ci-dessus.

La présente invention vise à fournir une composition cosmétique pour le traitement des cheveux, en particulier pour prolonger l'effet dans le temps de certains actifs cosmétiques compris dans la composition.

Ainsi, la présente invention concerne une composition cosmétique comprenant un milieu cosmétiquement acceptable, et :
a) au moins un polymère fixant et/ou au moins un agent épaississant,
b) un ou plusieurs types de capsules, chaque capsule comprenant une enveloppe imperméable constituée d'un ou plusieurs polymères, de préférence réticulés, lesdites capsules contenant un milieu liquide contenant au moins un actif ou constitué d'au moins un actif, le ou les actifs contenus dans un type de cellules étant différents du ou des actifs contenus dans un autre type de capsules, lesdites capsules contenant à titre d'actif(s) :
   b1) au moins un polymère fixant, ou
   b2) au moins un agent épaississant, ou
   b3) au moins ou un mono ou un poly alcool, ou
   b4) au moins un premier polymère réactif et au moins un deuxième polymère réactif pouvant réagir avec le ou lesdits premiers polymères réactifs, le ou lesdits premiers polymères réactifs étant isolés du ou des deuxièmes polymères réactifs,
lesdites capsules étant capables de libérer le ou les actifs qu'elles contiennent par rupture au moins partielle de l'enveloppe, la rupture de l'enveloppe pour chaque capsule pouvant s'effectuer aussi bien par l'action intentionnelle d'un utilisateur que sans action intentionnelle d'un utilisateur.

Par polymère, on entend au sens de la présente invention tout composé dont la structure comporte la répétition d'au moins un motif issu d'au moins un monomère. Ceci inclue les oligomères (nombre de répétitions inférieur à 10) et les polymères traditionnels (nombre de répétitions supérieur à 10).

Par polymère fixant, on entend au sens de la présente invention un polymère susceptible de conférer une forme à une chevelure, ou permettant de maintenir une forme donnée.

Le ou les polymères fixants compris dans la composition peuvent être choisis parmi les polymères fixants habituellement utilisés pour le coiffage des cheveux. Le ou les polymères fixants peuvent être choisis parmi les polymères anioniques, cationiques, non ioniques ou amphotères.

Les polymères anioniques généralement utilisés dans la présente invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 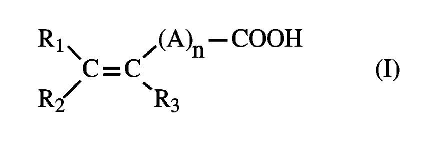 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène ou le soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂₋COOH, phényle ou benzyle.

Dans la formule (I) ci-dessus, un groupement alkyle inférieur comporte de préférence de 1 à 4 atomes de carbone et désigne en particulier, les groupements méthyle et éthyle.

Les polymères anioniques à groupements carboxyliques préférés selon l'invention sont :
A) les copolymères d'acide acrylique ou méthacrylique ou leurs sels, dont les copolymères d'acide acrylique et d'acrylamide et les copolymères acide méthacrylique/acide acrylique/acrylate d'éthyle/méthacrylate de méthyle, en particulier l'AMERHOLD DR 25 commercialisé par la société AMERCHOL, et les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371. On peut également citer les copolymères d'acide acrylique et de méthacrylate d'alkyle en C₁-C₄. On peut encore citer le terpolymère acrylate de butyle/acide acrylique/acide méthacrylique.
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
   On peut citer aussi comme copolymère dérivé d'acide crotonique les terpolymères acide crotonique/acétate de vinyle/ t.butylbenzoate de vinyle et en particulier le MEXOMERE PW fourni par la société CHIMEX.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.
F) Les polymères comprenant les groupements sulfoniques. Ces polymères peuvent être des polymères comportant des motifs vinylsulfonique, styrène-sulfonique, naphtalène-sulfonique, acrylamido-alkylsulfonique, sulfoisophtalates.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène-sulfonique, les sels de sodium, ayant une masse moléculaire d'environ 500 000 et d'environ 100 000. Ces composés sont décrits dans le brevet FR 2198719 ;
- les sels d'acides polyacrylamide-sulfoniques tels que ceux mentionnés dans le brevet US 4 128 631 ;
- les polymères siliconés greffés anioniques.

Les polymères siliconés greffés utilisés selon l'invention sont choisis préférentiellement parmi les polymères à squelette organique non-siliconé greffé par des monomères contenant un polysiloxane, les polymères à squelette polysiloxanique greffé par des monomères organiques non-siliconés et leurs mélanges.

Dans tout ce qui suit ou qui précède, on entend désigner par silicone ou polysiloxane, en conformité avec l'acceptation générale, tout polymère oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoralkyles, les radicaux aryles et en particulier phényle, et les radicaux alcényles et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalkylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites « organomodifiées »).

Dans ce qui suit, on entend désigner par « macromère polysiloxane » en conformité avec l'acceptation générale, tout monomère contenant dans sa structure une chaîne polymère du type polysiloxane.

Les polymères à squelette organique non-siliconé greffé par des monomères contenant un polysiloxane utilisé selon la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane conformes à la présente invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :
a) de 0 à 98 % en poids d'au moins un monomère (A) lipophile de faible polarité lipophile à insaturation éthylénique, polymérisable par voie radicalaire ;
b) de 0 à 98 % en poids d'au moins un monomère (B) hydrophile polaire à insaturation éthylénique, copolymérisable avec le ou les monomères du type (A) ;
c) de 0,01 à 50 % en poids d'au moins un macromère polysiloxane (C) de formule générale :

   X(Y)ₙSi(R)₃₋ₘZₘ (II)

   Où :
   X désigne un groupe vinylique copolymérisable avec les monomères (A) et (B) ;
   Y désigne un groupe de liaison divalent ;
   R désigne un hydrogène, un alkyle ou un alcoxy en C₁-C₆, un aryle C₆-C₁₂ ;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,963,935, US 4,728,571 et US 4,972,037 et les demandes de brevet EP-A-0412704, EP-A-0412707, EP-A-0640105. Ils ont un poids moléculaire moyen en nombre de préférence allant de 10 000 à 2 000 000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluoroalcanol ; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluoréther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluoroctane sulfoamido)éthylacrylate ; le 2-(N-butylperflurooctane sulfoamido)éthylacrylate et leurs mélanges.

On peut citer comme exemples de monomères polaires (B), l'acide acrylique, l'acide méthacrylique, l'acide maléique, l'anhydride maléique et leurs demi-esters.

Les macromères polysiloxane (C) de formule (I) préférentiels sont choisis parmi ceux répondant à la formule générale suivante (III) : 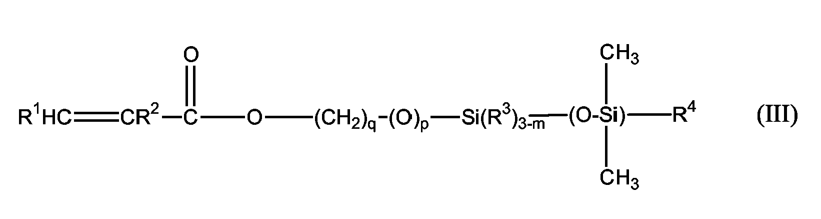 dans laquelle :
R¹ est l'hydrogène ou -COOH (de préférence hydrogène) ;
R² est l'hydrogène, méthyle ou -CH2COOH (de préférence méthyle) ;
R³ est un groupe alkyle, alcoxy ou alkylamino en C₁-C₆, aryle en C₁-C₁₂ ou hydroxyle (de préférence méthyle) ;
R⁴ est un groupe alkyle, alcoxy ou alkylamino en C₁-C₆, aryle en C₁-C₁₂ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1) ;

On utilise plus particulièrement les macromères polysiloxanes de formule : 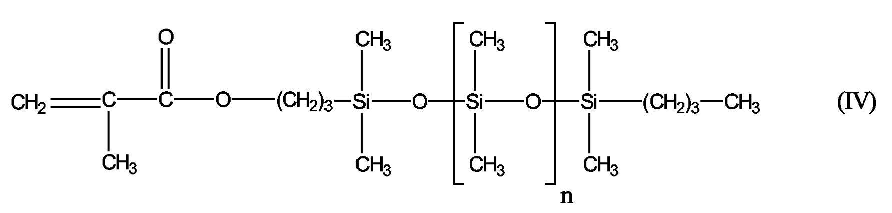 n étant un entier allant de 5 à 700.

Un mode particulier de réalisation de l'invention consiste à utiliser un copolymère susceptible d'être obtenu par polymérisation radicalaire à partir du mélange de monomères constitué par :
a) 60 % en poids d'acrylate de tertiobutyle :
b) 20 % en poids d'acide acrylique ;
c) 20 % en poids de macromère siliconé de formule : 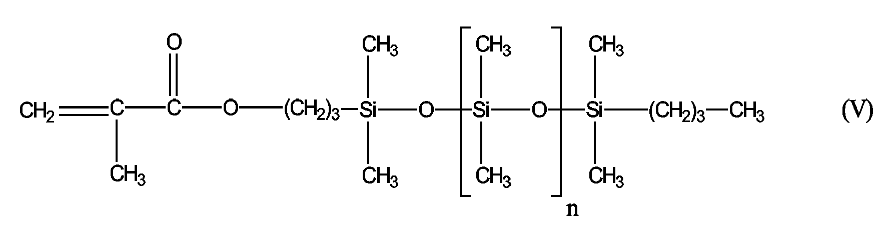 n étant un entier allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que le propylène, le styrène, les alkylstyrènes, le butylène, le butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (VI) :

T-(CH₂)ₛ-Si-[OSiR⁵R⁶)ₜ-R⁷]_{y} (VI)

dans laquelle T est choisi dans le groupe constitué par NH₂, NHR', une fonction époxy, OH, SH ; R⁵, R⁶, R⁷ et R', indépendamment, désignent un alkyle en C₁-C₆, phényle, benzyle, ou alkylphényle en en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5 000 à 300 000, plus préférentiellement de 8 000 à 200 000 et plus particulièrement de 9 000 à 40 000.

Selon la présente invention, le ou les polymères siliconés greffés, à squelette polysiloxanique greffé par des monomères organiques non-siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels-SH avec ces mêmes groupements vinyliques.

Des exemples de polymères à squelette polysiloxanique greffé par des monomères organiques non-siliconés convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (VII) suivant : 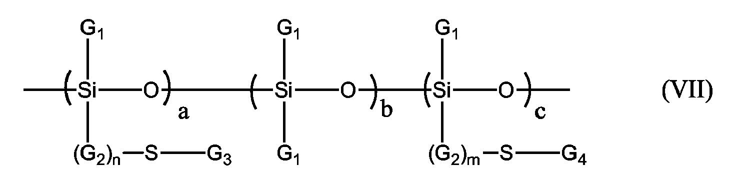 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkyle en C₁₋C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence le (méth)acrylate d'isobutyle ou de méthyle.

Des exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.
- les polyuréthanes.

Les polyuréthanes utilisés de préférence selon l'invention présentent de préférence un motif répétitif de base répondant à la formule (VIII) suivante :

-X'-B-X'-CO-NH-R-NH-CO- (VIII)

dans laquelle
- X' représente O et/ou NH,
- R est un radical divalent choisi parmi les radicaux alkylènes ramifiés ou non de type aromatique en C₆-C₂₀, aliphatique en C₁ à C₂₀, de préférence en C₁-C₆, cycloaliphatique en C₁ à C₂, de préférence en C₁-C₆, ces radicaux étant non substitués ou substitués par un ou plusieurs groupements halogène, alcoxy en C₁-C₄, aryle en C₆-C₃₀ en particulier phényle.
- le radical B est un radical divalent en C₁-C₃₀, de préférence C₂-C₁₀ et est porteur d'un groupement présentant une ou des fonctions carboxyliques et/ou une ou des fonctions sulfoniques, lesdites fonctions carboxyliques et/ou sulfoniques étant sous forme libre ou partiellement ou totalement neutralisées par une base minérale ou organique telle que les hydroxydes des métaux alcalins ou alcalinoterreux, l'ammoniaque et les alkylamines ou alcanolamines, les acides aminés organiques. De préférence B est le radical divalent issu de l'acide diméthylolpropionique.

Le radical R est avantageusement choisi parmi les radicaux répondant aux formules suivantes : 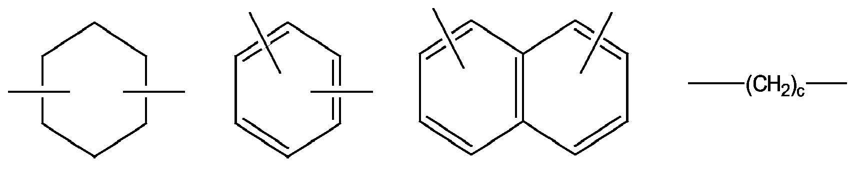 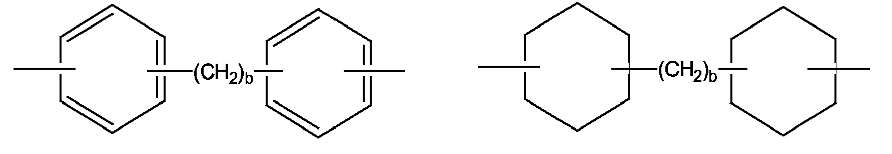 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4 et/ou 2,6-tolylène, 1,5-naphtylène, p-phénylène, méthylène-4,4bis-cyclohexyle et le radical divalent dérivé de l'isophorone.

Selon la présente invention, les polyuréthanes fixant peuvent comporter des greffons silicones et des silicones à greffons hydrocarbonés.

Un polyuréthane utilisé dans la présente invention peut avantageusement comprendre en outre au moins une séquence polysiloxane et son motif répétitif de base répond par exemple à la formule générale (IX) :

-X'-P-X'-CO-NH-R-NH-CO- (IX)

dans laquelle :
- P est un segment polysiloxanique,
- X' représente O et/ou NH, et
- R est un radical divalent choisi parmi les radicaux alkylènes ramifiés ou non de type aromatique en C₆-C₂₀, aliphatique en C₁₋C₂₀, de préférence en C₁-C₆, cycloaliphatique en C₁-C₂₀, de préférence en C₁-C₆, ces radicaux étant non substitués ou substitués par un ou plusieurs groupements halogène, alcoxy en C₁-C₄, aryle en C₁-C₃₀, en particulier phényle.

Le radical R est avantageusement choisi parmi les radicaux répondant aux formules suivantes : 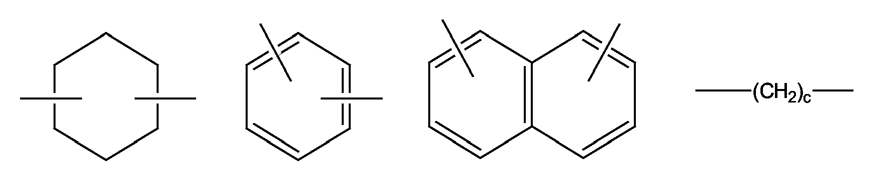 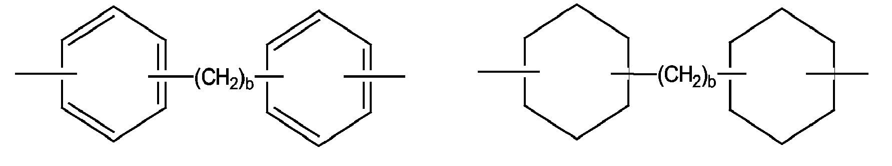 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4-et/ou 2,6-tolylène, 1,5-naphtylène, p-phénylène, méthylène-4,4bis-cyclohexyle et le radical divalent dérivé de l'isophorone.

Avantageusement, le segment polysiloxanique P répond à la formule générale (X) ci-après : 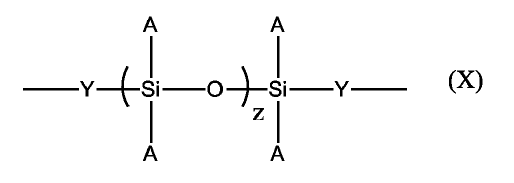 dans laquelle :
- les groupes A, qui peuvent être identiques ou différents, sont choisis parmi d'une part les groupes hydrocarbonés monovalents en C₁ à C₂₀, substantiellement exempts d'insaturation éthylénique et, d'autre part, les groupes aromatiques,
- Y représente un groupe hydrocarboné divalent, et
- Z représente un nombre entier, choisi de telle sorte que la masse moléculaire moyenne du segment polysiloxane soit comprise entre 300 et 10 000.

En général, le groupe divalent Y est choisi parmi les groupes alkylène de formule -(CH₂)ₐ-, dans laquelle a représente un nombre entier pouvant être compris entre 1 et 10.

Les groupes A peuvent être choisis parmi les groupes alkyle en C₁₋C₁₈, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle : les groupes arylalkyle, notamment benzyle et phényléthyle, ainsi que les groupes tolyle et xylyle.

A titre d'exemples de polyuréthane fixant, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset® PUR par la société BASF, le copolymère acide/diméthylol-propionique/isophorone-diisocyanate/néopentylglycol/polyesterdiols/dia-mine siliconée (connu aussi sous le nom de polyuréthane-6, appellation INCI) vendu sous la marque Luviset® Si PUR A par la société BASF.

On peut aussi utiliser des polyuréthanes anioniques élastomères.

Selon l'invention, les polymères anioniques sont de préférence choisis parmi les copolymères d'acide acrylique tels que le terpolymère acide acrylique/acrylate d'éthyle/N-tertiobutylacrylamide, en particulier celui vendu sous la dénomination ULTRAHOLD STRONG® par la société BASF, le terpolymère acrylate de butyle/acide acrylique/acide méthacrylique, en particulier celui vendu sous la dénomination Fixate G100 par la société NOVEON, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle, en particulier ceux vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléïque monoestérifié, en particulier celui vendu sous la dénomination GANTREZ® ES 425 par la société ISP, le LUVISET SI PUR, le LUVISET PUR, le MEXOMERE PW, les polyuréthanes anioniques, les silicones greffés anioniques et les polymères à groupements sulfoisophtalates, ainsi que l'AMERHOLD DR 25, le VS 80.

Les polymères amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n° 3 836 537.
   Le composé vinylique peut être également un sel de dialkyldiallylammonium tel que le chlorure de diéthyldiallyl-ammonium.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.
      Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide. le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/ acrylates/butylaminoethylmethacrylate copolymer tels que les produits vendus sous la dénomination AMPHOMER® ou LOVOCRYL® 47 par la société NATIONAL STARCH.
(3) les polyaminoamides réticulés et alkylés partiellement ou totalement dérivant de polyaminoamides de formule générale : 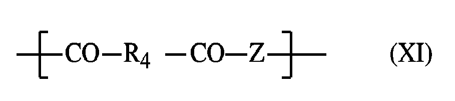 dans laquelle R₄ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 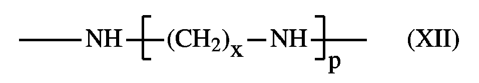 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylènediamine, ou le groupe dérivant de la pipérazine 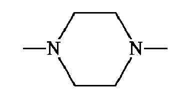
   c) dans les proportions de 0 à 20 % en moles, le groupe -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 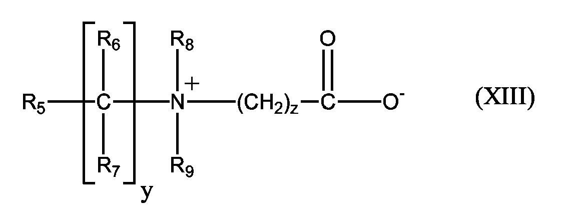 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent un atome d'hydrogène, un groupement méthyle, éthyle ou propyle, R₈ et R₉ représentent un atome d'hydrogène ou un groupe alkyle de telle façon que la somme des atomes de carbone dans R₁₀ et R₁₁ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
(5) les polymères dérivés du chitosane comportant des motifs monomères répondant aux formules suivantes: 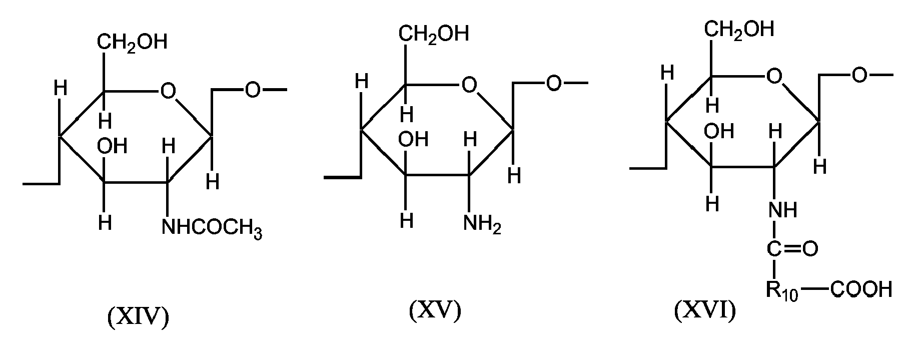 le motif (XIV) étant présent dans des proportions comprises entre 0 et 30 %, le motif (XV) dans des proportions comprises entre 5 et 50 % et le motif (XVI) dans des proportions comprises entre 30 et 90 %, étant entendu que dans ce motif F, R₁₀ représente un groupe de formule: 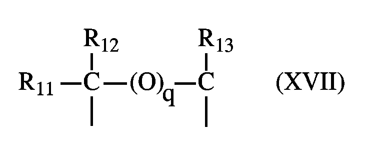 dans laquelle si q=0, R₁₁, R₁₂ et R₁₃, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane.
(7) Les polymères répondant à la formule générale (XVIII) décrits par exemple, dans le brevet français 1 400 366 constitués par l'enchaînement de motifs : 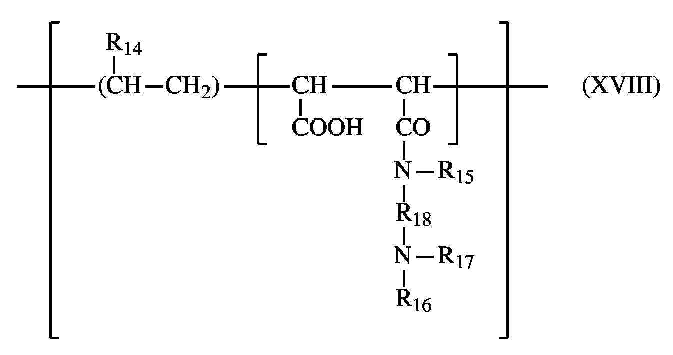 dans laquelle R₁₄ représente un atome d'hydrogène, un groupe CH₃O, CH₃CH₂O, phényle, R₁₅ désigne l'hydrogène ou un groupe alkyle inférieur tel que méthyle, éthyle, R₁₆ désigne l'hydrogène ou un groupe alkyle inférieur tel que méthyle, éthyle, R₁₇ désigne un groupe alkyle inférieur tel que méthyle, éthyle ou un groupe répondant à la formule: -R₁₈-N(R₁₆)₂, R₁₈ représentant un groupement -CH₂-CH₂-, -CH₂-CH₂-CH₂-, -CH₂-CH(CH₃)-, R₁₆ ayant les significations mentionnées ci-dessus, ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (XIX)

      où D désigne un groupe 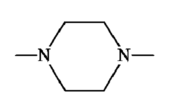 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XX)

      où D désigne un groupe 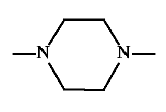 et X désigne le symbole E ou E' et au moins une fois E'; E ayant la signification indiquée ci-dessus et E' est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une
      ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.

Selon un mode de réalisation préféré de l'invention, les polymères amphotères fixants utilisables dans le procédé selon l'invention peuvent être choisis parmi les copolymères à blocs, ramifiés, comprenant :
(a) des motifs non ioniques dérivés d'au moins un monomère choisi parmi les (méth)acrylates d'alkyle en C₁-C₂₀, les N-mono-(alkyle en C₂-C₁₂)-(méth)acrylamides et les N,N-di-(alkyle en C₂-C₁₂)-(méth)acrylamide,
   (b) des motifs anioniques dérivés d'au moins un monomère choisi parmi l'acide acrylique et l'acide méthacrylique, et
   (c) des motifs polyfonctionnels dérivés d'au moins un monomère comportant au moins deux groupes fonctionnels insaturés polymérisables, et ayant de préférence une structure constituée de blocs hydrophobes sur lesquels sont fixés, par l'intermédiaire des motifs polyfonctionnels (c), plusieurs blocs plus hydrophiles.

Parmi les polymères amphotères, ceux de la famille (2) sont préférés et plus particulièrement parmi ceux-ci les produits vendus sous la dénomination AMPHOMER par National Starch.

Comme expliqué précédemment, le ou les polymères fixants peuvent encore être choisis parmi les polymères cationiques.

Par "polymère cationique", on entend tout polymère contenant des groupements cationiques et/ou des groupements ionisables en groupements cationiques.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont notamment décrits dans les brevets français FR 2 505 348 et FR 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français FR 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium ou de diméthyldiallylammonium ; on peut citer par exemple le polyquaternium 10 (nom INCI) ;
(4) les autres polysaccharides cationiques décrits plus particulièrement dans les brevets US 3 589 578 et US 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français FR 2 162 025 et FR 2 280 361 ;
(6) les polyaminoamides solubles dans l'eau, tels que ceux notamment décrits dans les brevets français FR 2 252 840 et FR 2 368 508 ;
(7) les dérivés de polyaminoamides, par exemple, les polymères acide adipique/dialkylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français FR 1 583 363 ;
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains US 3 227 615 et US 2 961 347 ;
(9) les cyclopolymères d'alkyldiallylamine ou de dialkyldiallylammonium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français FR 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020 ; on peut citer par exemple le chlorure d'hexadiméthrine (nom INCI), commercialisé par la société CHIMEX sous la référence MEXOMERE PO ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F;
(13) les polyamines comme le Polyquart® H vendu par HENKEL, référencées sous le nom de " POLYETHYLENEGLYCOL (15) TALLOW POLYAMINE " dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi le chlorure d'hexadiméthrine et les homo ou copolymères de chlorure de diméthyldiallylammonium.

Les polymères non ioniques utilisables selon la présente invention peuvent être choisis parmi :
- les homopolymères de vinylpyrrolidone ;
- les copolymères de vinylpyrrolidone et d'acétate de vinyle ;
- les polyalkyloxazolines telles que les polyéthyloxazolines commercialisées par la société DOW CHEMICAL sous les dénominations PEOX® 50 000, PEOX® 200 000 et PEOX® 500 000 ;
- les homopolymères d'acétate de vinyle tels que le produit proposé sous le nom de APPRETAN® EM par la société HOECHST ou le produit proposé sous le nom de RHODOPAS® A 012 par la société RHONE POULENC ;
- les copolymères d'acétate de vinyle et d'ester acrylique tels que le produit proposé sous le nom de RHODOPAS® AD 310 de RHONE POULENC ;
- les copolymères d'acétate de vinyle et d'éthylène comme le produit proposé sous le nom de APPRETAN® TV par la société HOECHST;
- les copolymères d'acétate de vinyle et d'ester maléique, par exemple de maléate de dibutyle tels que le produit proposé sous le nom de APPRETAN® MB EXTRA par la société HOECHST ;
- les copolymères de polyéthylène et d'anhydride maléique ;
- les homopolymères d'acrylates d'alkyle et les homopolymères de méthacrylates d'alkyle tels que le produit commercialisé sous la dénomination MICROPEARL® RQ 750 par la société MATSUMOTO ou le produit commercialisé sous la dénomination LUHYDRAN® A 848 S par la société BASF ;
- les copolymères d'esters acryliques tels que, par exemple, les copolymères d'acrylates d'alkyle et de méthacrylates d'alkyle comme les produits commercialisés par la société ROHM & HAAS sous les dénominations PRIMAL® AC-261 K et EUDRAGIT® NE 30 D, par la société BASF sous les dénominations ACRONAL® 601, LUHYDRAN® LR 8833 ou 8845, par la société HOECHST sous les dénominations APPRETAN® N 9213 ou N9212 ;
- les copolymères d'acrylonitrile et d'un monomère non ionique choisi, par exemple, parmi le butadiène et les (méth)acrylates d'alkyle; on peut citer les produits proposés sous les dénominations NIPOL® LX 531 8 par la société NIPPON ZEON ou ceux proposés sous la dénomination CJ 0601 8 par la société ROHM & HAAS ;
- les copolymères d'acétate d'alkyle et d'uréthanne tels que le produit 8538-33 par la société NATIONAL STARCH ;
- les polyamides tels que le produit ESTAPOR® LO 11 proposé par la société RHONE POULENC ;
- les gommes de guar non ioniques chimiquement modifiées ou non modifiées.

Les gommes de guar non ioniques non modifiées sont, par exemple, les produits vendus sous la dénomination VIDOGUM® GH 175 par la société UNIPECTINE et sous la dénomination JAGUAR® C par la société MEYHALL.

Les gommes de guar non ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont, par exemple, vendues sous les dénominations commerciales JAGUAR® HP8, JAGUAR® HP60 et JAGUAR® HP120, JAGUAR® DC 293 et JAGUAR® HP 105 par la société MEYHALL, ou sous la dénomination GALACTASOL® 4H4FD2 par la société AQUALON.

On peut aussi utiliser des polymères fixants non ioniques de type polyuréthane ou de type silicone greffé à squelette siloxanique ou hydrocarbone.

Comme expliqué précédemment, la composition cosmétique selon l'invention peut également comprendre un ou plusieurs agents épaississants.

Par agent épaississant, on entend tout composé organique ou minéral permettant d'accroître la viscosité du milieu final.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropylguar, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société ALLIED COLLOIDS, ACULYN 22, 28, 33, 44 ou 46 par la société ROHM & HAAS et ELFACOS T210 et T212 par la société AKZO.

Comme expliqué précédemment, la composition cosmétique selon l'invention comprend un ou plusieurs types de capsules. Chaque capsule comprend une enveloppe imperméable constituée d'un ou plusieurs polymères de préférence réticulés.

Le ou les polymères constituant l'enveloppe des capsules peuvent être choisis parmi les résines urée/aldéhydes, les résines phénol/aldéhydes, les résines acryliques, les résines polyuréthanes, les résines polyesters, les résines polyamides, les polysaccharides, les résines mélamine aldéhyde.

Les capsules utilisées dans la composition selon l'invention ont généralement une taille comprise entre 1 et 100 µm, de préférence entre 5 et 50 µm.

L'enveloppe des capsules a généralement une épaisseur comprise entre 1 nm et 50 µm.

Les capsules présentes dans la composition selon l'invention contiennent un milieu liquide contenant au moins un actif liquide ou constitué d'au moins un actif. Ainsi, le ou les actifs peuvent constituer en eux-mêmes le milieu liquide, ou bien être contenus dans un milieu liquide classiquement utilisé dans les compositions cosmétiques.

Lorsqu'une capsule contient un actif unique, elle est de préférence univacuolaire.

Par capsule univacuolaire, on entend une capsule dont l'enveloppe ne renferme qu'un seul compartiment.

Lorsqu'une capsule contient plusieurs actifs, elle est de préférence multivacuolaire.

Par capsule multivacuolaire, on entend une capsule dont l'enveloppe renferme une pluralité de compartiments séparés par des parois de même nature chimique que l'enveloppe.

Selon un autre mode de réalisation, les capsules peuvent elles mêmes contenir des capsules secondaires. Dans ce cas, les capsules secondaires sont de plus petite taille, généralement de taille nanométrique.

Comme expliqué précédemment, les capsules présentes dans la composition selon l'invention contiennent à titre d'actif(s) :
b1) au moins un polymère fixant, ou
b2) au moins un polymère épaississant, ou
b3) au moins un mono ou polyalcool, ou
b4) au moins un premier polymère réactif et au moins un deuxième polymère pouvant réagir avec le ou lesdits premiers polymères, le ou lesdits premiers polymères étant isolés du ou des deuxièmes polymères.

Le ou les polymères fixants et le ou les polymères épaississants pouvant être contenus dans les capsules peuvent être choisis respectivement parmi tous les polymères fixants et polymères épaississants décrits précédemment.

Par monoalcool, on entend au sens de la présente invention un composé organique contenant de 1 à 100 et de préférence de 1 à 20 atomes de carbone et une seule fonction alcool. De préférence, le monoalcool ne contient pas de fonction éther. Avantageusement, le monoalcool sera choisi parmi l'éthanol, l'isopropanol, le tertiobutanol.

Par polyalcool, on entend au sens de la présente invention un composé organique contenant de 2 à 100 et de préférence de 2 à 20 atomes de carbone et de préférence de 3 à 20 atomes de carbone et comportant de 2 à 10 fonctions alcools.

Avantageusement, le polyalcool est choisi parmi le propylène alcool, le glycérol, le néopentylglycol, l'isoprène glycol, les 2 méthyl, 1,3-propanediol , le pinacol, l'hexylène glycol, le dipropylène glycol, le dipropylène glycol, le 1,5 pentanediol, le 3-méthyl 1,5 pentanediol, les polyéthylène glycols.

Le ou les premiers polymères réactifs pouvant être contenus dans les capsules peuvent présenter une ou plusieurs fonctions chimiques A' choisies parmi les fonctions suivantes :
- époxyde,
- aziridine,
- vinyle et vinyle activée, en particulier, acrylonitrile, esters acrylique et métacrylique, acide et esters crotonique, acide et esters cinnamique, styrène et dérivés, butadiène, éthers de vinyle, vinylcétone, esters maléiques, vinyl sulfones, maléimides,
- anhydride, chlorure d'acide et esters d'acide carboxylique,
- aldéhydes,
- acétals, hémi-acétals,
- aminals, hémi aminals,
- cétones, alpha-hydroxycétones, alpha-halocétones,
- lactones, thiolactones,
- isocyanate,
- thiocyanate,
- imines,
- imides, en particulier, succinimide, glutimide,
- N-hydroxysuccinimide esters,
- imidates,
- thiosulfate,
- oxazine et oxazoline,
- oxazinium et oxazolinium,
- halogénures d'alkyle en C₁ à C₃₀ ou d'aryle ou aralkyle en C₆ à C₃₀ de formule RX avec X = I, Br, Cl,
- halogénure de cycle insaturé, carboné ou hétérocycle, notamment les chlorotriazine, chloropyrimidine, chloroquiinoxaline, chlorobenzotriazole,
- halogénure de sulfonyle de formule RSO₂Cl ou F, R étant un alkyle en C₁ à C₃₀.

De préférence, les fonctions chimiques A' sont choisies parmi les fonctions anhydride, époxyde, chlorotriazine, aldéhyde, thiosulfate.

Le ou les deuxièmes polymères réactifs pouvant être contenus dans les capsules peuvent présenter une ou plusieurs fonctions chimiques B' choisies parmi les fonctions hydroxy, amines primaires et secondaires, les thiols, les acides carboxyliques.

Les premiers et deuxièmes polymères réactifs utilisés selon l'invention peuvent contenir des fonctions chimiques autres que les fonctions A' et B'.

Les premiers et deuxièmes polymères réactifs peuvent être d'origine naturelle, modifiés chimiquement ou non, comme par exemple les polysaccharides tels que la cellulose, le dextrane, le chitosane, le guar et leurs dérivés hydroxyalkylés, carboxyméthylés, aminés ou thiolés, ou leurs dérivés à fonction aldéhyde ou époxy.

Ils peuvent être des polymères synthétiques tels que les polyacrylates, les polyméthacrylates, les polyvinyles, les polyesters, les polyéthers, les polyamides, les polyuréthanes, les polydiméthylsiloxanes, les polypeptides. Ces polymères peuvent être synthétisés selon des méthodes connues dans la littérature.

Ces polymères réactifs peuvent se présenter sous tout type de topologie : chaîne linéaire, ramifiée, en étoile ou hyperbranchée, tels que les dendrimères, chaînes séquencées, statistiques ou alternées.

Les fonctions chimiques A' et B' peuvent être présentes sur la chaîne polymère, en bout de chaîne, greffées le long de la chaîne principale ou des chaînes secondaires, sur les branches des polymères en étoile ou hyperbranchés.

Dans un mode de réalisation particulier de l'invention, le ou les polymères réactifs possèdent au moins deux fonctions chimiques A' identiques et le ou les deuxièmes polymères réactifs possèdent au moins deux fonctions chimiques B' identiques, afin de se lier avec au moins deux autres polymères.

De préférence, le ou les premiers polymères réactifs sont choisis parmi les polymères suivants :
- les polymères contenant le motif anhydride, notamment maléique,
- les polymères contenant le groupe époxyde et / ou aldéhyde,
- les polysaccharides naturels ou modifiés présentant des fonctions aldéhydes,
- les polysaccharides naturels ou modifiés présentant des fonctions époxy,
- les polymères naturels ou synthétiques à fonctions acide carboxylique.

Le ou les deuxièmes polymères réactifs sont de préférence choisis parmi les polymères synthétiques ou naturels à fonctions OH ou NH₂ ou SH ou COOH.

A titre d'exemples de polymères synthétiques ou naturels à fonctions OH ou NH₂ ou SH ou COOH, on peut citer :
- les dendrimères à terminaisons OH, ou NH₂ ou SH ou COOH de une ou plusieurs générations,
- les polymères synthétiques à fonction hydroxyle, tes que les alcools polyvinyliques,
- les polyéthylèneimines,
- les polyéthylèneimine thiols, obtenus par réaction de polyéthylèneimines sur la γ-butyrolactone, tels que ceux décrits dans le brevet L'OREAL FR 2 772 770,
- les polyacides aminés présentant des groupes OH, ou NH₂ ou SH ou COOH libres, par exemple la polylysine,
- les polysaccharides naturels ou modifiés présentant des fonctions OH, ou NH₂ ou SH ou COOH, comme par exemple le chitosane et ses dérivés, tels que les carboxyméthyl-chitosanes ou les amino-dextranes.

Selon la présente invention, les associations dans les capsules de premiers et deuxièmes polymères réactifs suivantes sont préférées :
- un deuxième polymère réactif choisi parmi les dendrimères à terminaisons OH, ou NH₂ ou SH ou COOH de une ou plusieurs générations en association avec un premier polymère réactif choisi parmi les polymères contenant le motif anhydride, notamment maléique, ou les polymères contenant le groupe époxyde et / ou aldéhyde,
- un deuxième polymère réactif choisi parmi les polymères synthétiques à fonction hydroxyle, tels les alcools polyvinyliques, en association avec un premier polymère réactif choisi parmi les polymères contenant le motif anhydride, notamment maléique ou les polymères contenant le groupe époxyde,
- un deuxième polymère réactif choisi parmi les polyéthylèneimines en association avec un premier polymère réactif choisi parmi les polymères contenant le motif anhydride, notamment maléique ou les polymères contenant le groupe époxyde et / ou aldéhyde,
- un deuxième polymère réactif choisi parmi les polyéthylèneimine thiols, obtenus par réaction de polyéthylèneimines sur la γ-butyrolactone, tels que ceux décrits dans le brevet L'OREAL FR 2 772 770, en association avec un premier polymère réactif choisi parmi les polymères contenant le motif anhydride, notamment maléique ou les polymères contenant le groupe époxyde et / ou aldéhyde,
- un deuxième polymère réactif choisi parmi les polyacides aminés présentant des groupes OH, ou NH₂ ou SH ou COOH libres, par exemple la polylysine en association avec un premier polymère réactif choisi parmi les polymères contenant le motif anhydride, notamment maléique ou les polymères contenant le groupe époxyde et / ou aldéhyde,
- un deuxième polymère réactif choisi parmi les polysaccharides naturels ou modifiés présentant des fonctions OH, ou NH₂ ou SH ou COOH en association avec un premier polymère réactif choisi parmi les polymères contenant le motif anhydride, notamment maléique ou les polymères contenant le groupe époxyde et / ou aldéhyde,
- un premier polymère réactif choisi parmi les polysaccharides naturels ou modifiés présentant des fonctions aldéhydes en association avec un deuxième polymère réactif choisi parmi les polymères synthétiques ou naturels à fonctions OH ou NH₂ ou SH ou COOH comme par exemple la polyéthylèneimine, la polylysine, le chitosane et ses dérivés, tels que les carboxyméthyl-chitosanes ou les amino-dextranes,
- un premier polymère réactif choisi parmi les polysaccharides naturels ou modifiés présentant des fonctions époxy en association avec un deuxième polymère réactif choisi parmi les polymères synthétiques ou naturels à fonctions OH ou NH₂ ou SH ou COOH comme par exemple la polyéthylèneimine, la polylysine, le chitosane et ses dérivés, tels que les carboxyméthyl-chitosanes ou les amino-dextranes,
- un premier polymère réactif choisi parmi les polymères naturels ou synthétiques à fonctions acide carboxylique en association avec un deuxième polymère réactif choisi parmi les polymères synthétiques ou naturels à fonctions OH, NH₂ ou SH, en présence d'un carbodiimide, ou d'un acide, d'une base ou d'une enzyme, comme les estérase, lipase, protéase.

Par exemple, et sans limitation, on peut citer, à titre de premiers polymères réactifs, les polymères énumérés ci-après :
- Copolymère méthyl-vinyl éther / anhydride maléique, en particulier commercialisé par exemple par ISP sous le nom de Gantrez,
- PolyMethacrylate de glycidyle, en particulier commercialisé par Polysciences,
- Polydimethylsiloxane de glycidyle, en particulier commercialisé par Shinetsu (référence X-2Z-173 FX ou DX),
- Polyamidoamine époxy, par exemple commercialisé par Hercules sous le nom de Delsette 101, le Kymène 450 de chez Hercules,
- Epoxy-dextrane,
- Polysaccharides polyaldéhydes obtenus par oxydation de polysaccharides par NalO4 (méthodes connues, Bioconjugate Techniques ; Hermanson GT, Academic Press, 1996),
   et, à titre de polymères réactifs, les polymères énumérés ci-après :
- Dendrimère PAMAM, en particulier commercialisé par Dendritech, DSM, Sigma-Aldrich (STARBURST, PAMAM DENDRIMER, G(2, O) de chez DENDRITECH),
- Dendrimère à fonctions hydroxy, en particulier commercialisé par Perstorp, DSM, (exemple : HBP TMP core 2 Generation PERSTORP),
- PEI (polyéthylène-imine), en particulier commercialisé par BASF, sous le nom de Lupasol,
- PEI-Thiol,
- Polylysine, en particulier commercialisé par Chisso,
- HP cellulose, tel que KLUCELEF de chez AQUALON,
- Amino-dextrane, par exemple commercialisé par Carbomer,
- Amino-cellulose, par exemple ceux décrits dans WO 01/25283 de BASF,
- PVA (polyvinylacétal), par exemple AIRVOL 540 de chez AIRPRODUCTS CHEMICAL,
- Amino PVA, par exemple commercialisé par Carbomer,
- Chitosane,
- CM ou HP Dextrane, par exemple commercialisé par Fluka,
- CM ou HP Chitosane, par exemple commercialisé par Fluka.

Comme expliqué précédemment, le ou les premiers polymères réactifs sont isolés du ou des deuxièmes polymères réactifs.

Ainsi, le ou les premiers polymères réactifs et le ou les deuxièmes polymères réactifs peuvent être contenus dans des capsules de types différents, ou bien dans un même type de capsules. Dans ce dernier cas, les capsules sont multivacuolaires.

Les capsules décrites ci-dessus présentes dans la composition représentent généralement de 0,5 à 70%, de préférence de 1 à 50%, en poids par rapport au poids total de la composition.

Selon un mode de réalisation particulier de l'invention, la composition selon l'invention peut comprendre en outre des capsules supplémentaires contenant au moins un solvant autre qu'un mono ou polyalcool, et/ou des capsules supplémentaires contenant au moins un plastifiant, et/ou des capsules supplémentaires contenant des particules associées à un polymère.

Les capsules supplémentaires pouvant être comprises dans la composition selon l'invention sont comparables aux capsules décrites précédemment et contenant un milieu liquide contenant au moins un actif ou constituées d'au moins un actif.

En outre, les capsules supplémentaires sont capables de libérer le ou les actifs qu'elle contiennent par rupture au moins partielle de l'enveloppe, la rupture de l'enveloppe pour chaque capsule pouvant s'effectuer aussi bien par l'action intentionnelle d'un utilisateur que sans action intentionnelle d'un utilisateur.

Le ou les solvants pouvant être contenus dans les capsules supplémentaires peuvent être choisis parmi les cétones en C₃-C₆, de préférence l'acétone, la méthyléthylcétone, les esters en C₃-C₆, de préférence l'acétate de méthyle, l'acétate de butyle, l'acétate d'éthyle, les éthers en C₃-C₆, de préférence le diméthoxyéthane, le diéthoxyéthane.

Les capsules supplémentaires représentent généralement de 0,1 à 50% en poids, de préférence 0,5 à 30% en poids total de la composition.

Comme expliqué précédemment, la composition cosmétique selon l'invention comprend un milieu cosmétiquement acceptable.

Généralement, le milieu cosmétiquement acceptable est constitué par l'eau, les alcools en C₁-C₄, de préférence l'éthanol ou l'isopropanol, les cétones en C₃-C₆, de préférence l'acétone, la méthyléthylcétone, les esters en C₃-C₆, de préférence l'acétate de méthyle, l'acétate de butyle, l'acétate d'éthyle, les éthers en C₃-C₆, de préférence le diméthoxyéthane, le diéthoxyéthane, les alcools gras, les polyols modifiés ou non, de préférence le glycérol, les glycols, les silicones volatiles ou non, les huiles minérales organiques ou végétales, les cires, les acides gras, ou leurs mélanges.

Le milieu cosmétiquement acceptable peut se présenter sous la forme d'une solution ou d'une émulsion.

La composition cosmétique selon l'invention peut également comprendre un ou plusieurs additifs cosmétiques classiquement utilisés dans les compositions cosmétiques. On peut citer en particulier les silicones, sous forme soluble, dispersée, micro ou nano-dispersée, les agents tensioactifs non-ioniques, anioniques, cationiques ou amphotères, les agents conditionneurs, les agents adoussissants, les agents anti-mousse, les agents hydratants, les agents émollients, les plastifiants, les filtres solaires hydrosolubles ou liposolubles, siliconés ou non, les colorants permanents ou temporaires, les pigments minéraux ou organiques, colorés ou non colorés, les charges minérales, les argiles, les nacres, les opacifiants, les colloïdaux, les parfums, les peptisants, les conservateurs, les céramides et pseudo-céramides, les vitamines et les provitamines, par exemple le panthénol, les protéines, les agents séquestrants, les agents solubilisants, les agents alcalinisants, les agents anti-corrosion, les corps gras tels que les huiles végétales, animales, minérales et synthétiques, les agents réducteurs ou antioxydants, les agents oxydants, et leurs mélanges.

La composition selon l'invention peut se présenter sous la forme d'une lotion, d'une mousse, d'une crème, d'une cire ou d'un gel.

La composition selon l'invention peut être conditionnée dans un dispositif aérosol. Dans ce cas, elle comprend au moins un agent propulseur.

En général, l'agent propulseur est constitué par un gaz comprimé ou liquéfié choisi parmi l'air, le gaz carbonique, l'azote comprimé, un gaz soluble, en particulier le diméthyléther, les hydrocarbures volatils, en particulier le n-butane, le propane, l'isobutane, le pentane, les hydrocarbures halogénés, fluorés ou non, et leurs mélanges.

L'invention concerne encore un procédé pour le traitement cosmétique des cheveux comprenant l'application sur les cheveux d'une composition cosmétique selon l'invention, suivie d'une rupture des capsules en une ou plusieurs étapes.

Selon un mode de réalisation préféré, le procédé selon l'invention permet de maintenir dans le temps la mise en forme des cheveux, et comprend les étapes suivantes :
1) application sur les cheveux d'une composition cosmétique telle que définie précédemment et contenant au moins un polymère fixant et comprenant à titre d'actif(s) encapsulé(s) au moins un polymère fixant.
2) rupture de l'enveloppe des capsules en une ou plusieurs étapes.

Avantageusement, la composition cosmétique comprend des capsules supplémentaires contenant au moins un solvant.

La composition cosmétique appliquée sur les cheveux permet d'effectuer la mise en forme des cheveux.

Après un certain temps, la rupture de l'enveloppe des capsules peut se faire en une ou plusieurs étapes lorsque l'effet fixant du à la présence du ou des polymères fixants commence à diminuer, notamment lorsque le film formé par le ou les polymères extérieurs se craquelle.

La présence dans la composition de capsules contenant un solvant permet, lorsque le solvant est libéré, de redissoudre le ou les polymères fixant et/ou le ou les agents épaississants séchés sur les cheveux et de les resolubiliser pour effectuer une nouvelle mise en forme des cheveux ou maintenir dans le temps la mise en forme initiale des cheveux.

La rupture de l'enveloppe des capsules peut se faire par action intentionnelle d'un utilisateur, de préférence par brossage ou friction des cheveux, par exposition à un stimulus, tel que la chaleur, un rayonnement UV, ou par application d'une solution, telle qu'un solvant ou une solution saline.

Lorsque la rupture de l'enveloppe des capsules se fait par exposition à la chaleur, la température utilisée est de préférence comprise entre 40°C et 200°C.

Lorsque la rupture de l'enveloppe des capsules se fait par application d'un solvant, le solvant utilisé peut être un solvant hydroalcoolique, l'eau ou une solution saline. Le solvant permet ainsi de dissoudre l'enveloppe des capsules.

La rupture de l'enveloppe des capsules peut également se faire sans action intentionnelle d'un utilisateur, par exemple par érosion de l'enveloppe, par rupture sous la contrainte du film formé par la composition, par action de la chaleur ou d'un rayonnement UV.

Le mode de rupture de l'enveloppe des capsules est indépendant du type de capsules. Pour chaque type de capsule, l'enveloppe peut se rompre aussi bien par l'action intentionnelle d'un utilisateur que sans l'action intentionnelle d'un utilisateur.

La présente invention est illustrée par les exemples suivants.

### Exemple 1 Composition pour effectuer une mise en forme des cheveux postérieurement à une première mise en forme

On applique 1 g d'une composition selon l'invention, sous forme de gel, sur 2,5 g de cheveux européens.

La composition présente la formulation suivante :

| | |
|---|---|
| Fixate G100 (terpolymère acrylate de butyle/acide acrylique/acide méthacrylique) | 3,0% |
| Carbomer | 0,5% |
| Capsules contenant un polymère acrylique | 5,0% |
| Eau | 91,5% |

Une synthèse analogue à celle des capsules utilisées dans la composition de l'exemple 1 est décrite dans la publication Brown, Kessler, Sottos, White, Journal of Microencapsulation, 20(2003) 719-730.

La composition est appliquée sur cheveux secs. On laisse sécher à l'air libre. On obtient une première mise en forme des cheveux.

Lorsque l'utilisateur souhaite effectuer une nouvelle mise en forme des cheveux, il lui suffit de frictionner les mèches de cheveux. Cela permet la libération du polymère contenu dans les capsules et ainsi de fixer à nouveau la coiffure dans la nouvelle forme voulue.

### Exemple 2 Composition pour effectuer une mise en forme des cheveux postérieurement à une première mise en forme

On applique 1 g d'une composition selon l'invention, sous forme de gel, sur 2,5 g de cheveux européens.

La composition présente la formulation suivante :

| | |
|---|---|
| Fixate G100 | 3,0% |
| Carbomer | 0,5% |
| Capsules contenant un polymère acrylique | 5,0% |
| Capsules contenant de l'éthanol | 5,0% |
| Eau | 86% |

Une synthèse de capsules analogues à celle des capsules contenant un polymère acrylique utilisées dans la composition de l'exemple 2 est décrite dans la publication Brown, Kessler, Sottos, White, Journal of Microencapsulation, 20(2003) 719-730.

La composition est appliquée sur cheveux secs. On laisse sécher à l'air libre. On obtient une première mise en forme des cheveux.

Lorsque l'utilisateur souhaite effectuer une nouvelle mise en forme des cheveux, il lui suffit de frictionner les mèches de cheveux. Cela permet la libération du polymère et du solvant contenu dans les capsules et ainsi de fixer à nouveau la coiffure dans la nouvelle forme voulue. Le solvant permet de dissoudre le polymère déjà sec sur les cheveux et de faciliter l'étalement du polymère contenu dans les capsules.

## Revendications

1. Composition cosmétique **caractérisée en ce qu'**elle comprend un milieu cosmétiquement acceptable, et :
a) au moins un polymère fixant et/ou au moins un agent épaississant,
b) un ou plusieurs types de capsules, chaque capsule comprenant une enveloppe imperméable constituée d'un ou plusieurs polymères, de préférence réticulés, lesdites capsules contenant un milieu liquide contenant au moins un actif liquide ou constitué d'au moins un actif, le ou les actifs contenus dans un type de cellules étant différents du ou des actifs contenus dans un autre type de capsules, lesdites capsules contenant à titre d'actif(s) :
b1) au moins un polymère fixant, ou
b2) au moins un polymère épaississant, ou
b3) au moins un mono ou polyalcool, ou
b4) au moins un premier polymère réactif et au moins un deuxième polymère réactif pouvant réagir avec le ou lesdits premiers polymères réactifs, le ou lesdits premiers polymères réactifs étant isolés du ou des deuxièmes polymères réactifs,
lesdites capsules étant capables de libérer le ou les actifs qu'elle contiennent par rupture au moins partielle de l'enveloppe, la rupture de l'enveloppe pour chaque capsule pouvant s'effectuer aussi bien par l'action intentionnelle d'un utilisateur que sans action intentionnelle d'un utilisateur.

2. Composition cosmétique selon la revendication 1 **caractérisée en ce que** le ou les polymères fixants sont choisis parmi les polymères fixants anioniques, non anioniques, cationiques ou amphotères.

3. Composition cosmétique selon la revendication 2 **caractérisée en ce que** le ou les polymères fixants sont choisis parmi les copolymères d'acide acrylique tels que le terpolymère acide acrylique/acrylate d'éthyle/N-tertiobutylacrylamide, le terpolymère acrylate de butyle/acide acrylique/ acide méthacrylique, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther / anhydride maléïque monoestérifié, les polyuréthanes anioniques, les silicones greffés anioniques et les polymères à groupements sulfoisophtalates.

4. Composition cosmétique selon l'une quelconque des revendications précédentes **caractérisée en ce que** le ou les polymères fixants sont choisis parmi les polymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

5. Composition cosmétique selon l'une quelconque des revendications précédentes **caractérisée en ce que** le ou les polymères fixants sont choisis parmi :
- les homopolymères de vinylpyrrolidone ;
- les copolymères de vinylpyrrolidone et d'acétate de vinyle.

6. Composition cosmétique selon l'une quelconque des revendications précédentes **caractérisée en ce que** le ou les agents épaississants sont choisis parmi les agents épaississants cellulosiques, par exemple l'hydroxyéthylcellulose, l'hydroxypropylcellulose et le carboxypropylcellulose, la gomme de guar et ses dérivés, par exemple l'hydroxypropylguar, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères.

7. Composition cosmétique selon l'une quelconque des revendications précédentes **caractérisée en ce que** le ou les polymères constituant l'enveloppe imperméable des capsules sont choisis parmi les résines urée/aldéhydes, les résines phénol/aldéhydes, les résines acryliques, les résines polyuréthanes, les résines polyesters, les résines polyamides, les polysaccharides, les résines mélamine aldéhydes.

8. Composition cosmétique selon l'une quelconque des revendications précédentes **caractérisée en ce que** les capsules ont une taille comprise entre 1 et 100 µm, de préférence entre 5 et 50 µm.

9. Composition cosmétique selon l'une quelconque des revendications précédentes **caractérisée en ce que** l'enveloppe des capsules a une épaisseur comprise entre 1 nm et 45 µm.

10. Composition cosmétique selon l'une quelconque des revendications précédentes **caractérisée en ce que** les capsules sont univacuolaires ou multivacuolaires.

11. Composition cosmétique selon l'une quelconque des revendications précédentes **caractérisée en ce que** les capsules représentent de 0,5 à 70%, de préférence de 1 à 50%, en poids par rapport au poids total de la composition.

12. Composition cosmétique selon l'une quelconque des revendications précédentes **caractérisée en ce qu'**elle comprend en outre des capsules supplémentaires contenant au moins un solvant autre qu'un mono ou polyalcool, et/ou des capsules supplémentaires contenant au moins un agent épaississant, et/ou des capsules supplémentaires contenant au moins un plastifiant, et/ou des capsules supplémentaires contenant des particules associées à un polymère, lesdites capsules supplémentaires comprenant une enveloppe imperméable constituée d'un ou plusieurs polymères, de préférence réticulés, lesdites capsules supplémentaires étant capables de libérer le ou les composés qu'elle contiennent par rupture au moins partielle de l'enveloppe, la rupture de l'enveloppe pour chaque capsule supplémentaire pouvant s'effectuer aussi bien par l'action intentionnelle d'un utilisateur que sans action intentionnelle d'un utilisateur.

13. Composition cosmétique selon la revendication 12 **caractérisée en ce que** le ou les solvants contenus dans les capsules supplémentaires sont choisis parmi les cétones en C₃-C₆, de préférence l'acétone, la méthyléthylcétone, les esters en C₃-C₆, de préférence l'acétate de méthyle, l'acétate de butyle, l'acétate d'éthyle, et les éthers en C₃-C₆, de préférence le diméthoxyéthane et le diéthoxyéthane.

14. Composition cosmétique selon l'une quelconque des revendications 12 et 13 **caractérisée en ce que** les capsules supplémentaires représentent de 0,1 à 50%, de préférence de 0,5 à 30%, en poids du poids total de la composition.

15. Composition cosmétique selon l'une quelconque des revendications précédentes **caractérisée en ce que** le milieu cosmétiquement acceptable est constitué par l'eau, les alcools en C₁-C₄, de préférence l'éthanol ou l'isopropanol, les cétones en C₃-C₆, de préférence l'acétone, la méthyléthylcétone, les esters en C₃-C₆, de préférence l'acétate de méthyle, l'acétate de butyle, l'acétate d'éthyle, les éthers en C₃-C₆, de préférence le diméthoxyéthane, le diéthoxyéthane, les alcools gras, les polyols modifiés ou non, de préférence le glycérol, les glycols, les silicones volatiles ou non, les huiles minérales organiques ou végétales, les cires, les acides gras, ou leurs mélanges.

16. Composition cosmétique selon l'une quelconque des revendications précédentes **caractérisée en ce que** le milieu cosmétiquement acceptable se présente sous forme d'une solution ou sous forme d'une émulsion.

17. Composition cosmétique selon l'une quelconque des revendications précédentes **caractérisée en ce qu'**elle comprend en outre un ou plusieurs additifs cosmétiques choisis parmi les silicones, sous forme soluble, dispersée, micro ou nano-dispersée, les agents tensioactifs non-ioniques, anioniques, cationiques ou amphotères, les agents conditionneurs, les agents adoussissants, les agents anti-mousse, les agents hydratants, les agents émollients, les plastifiants, les filtres solaires hydrosolubles ou liposolubles, siliconés ou non, les colorants permanents ou temporaires, les pigments minéraux ou organiques, colorés ou non colorés, les charges minérales, les argiles, les nacres, les opacifiants, les colloïdaux, les parfums, les peptisants, les conservateurs, les céramides et pseudo-céramides, les vitamines et les provitamines, par exemple le panthénol, les protéines, les agents séquestrants, les agents solubilisants, les agents alcalinisants, les agents anti-corrosion, les corps gras tels que les huiles végétales, animales, minérales et synthétiques, les agents réducteurs ou antioxydants, les agents oxydants, et leurs mélanges.

18. Composition cosmétique selon l'une quelconque des revendications précédentes **caractérisée en ce qu'**elle se présente sous la forme d'une lotion, d'une mousse, d'une crème, d'une cire ou d'un gel.

19. Composition cosmétique selon l'une quelconque des revendications précédentes **caractérisée en ce qu'**elle comprend un agent propulseur.

20. Composition cosmétique selon la revendication 19 **caractérisée en ce que** l'agent propulseur est constitué par un gaz comprimé ou liquéfié choisi parmi l'air, le gaz carbonique, l'azote comprimé, un gaz soluble en particulier le diméthyléther, les hydrocarbures volatils, en particulier le n-butane, le propane, l'isobutane, le pentane, les hydrocarbures halogénés, fluorés ou non, et leurs mélanges.

21. Procédé pour le traitement cosmétique des cheveux comprenant l'application sur les cheveux d'une composition cosmétique telle que définie à l'une quelconque des revendications précédentes, suivie d'une rupture des capsules en une ou plusieurs étapes.

22. Procédé selon la revendication 21 pour maintenir dans le temps la mise en forme des cheveux, **caractérisé en ce qu'**il comprend les étapes suivantes :
1) application sur les cheveux d'une composition cosmétique telle que définie à l'une quelconque des revendications 1 à 20 comprenant un polymère fixant et comprenant à titre d'actif(s) encapsulé(s) au moins un polymère fixant,
2) rupture des capsules en une ou plusieurs étapes.

23. Procédé selon la revendication 22 **caractérisé en ce que** la composition cosmétique comprend des capsules supplémentaires contenant au moins un solvant.

24. Procédé selon l'une quelconque des revendications 21 à 23 **caractérisé en ce que** la rupture de l'enveloppe des capsules se fait par action intentionnelle d'un utilisateur, de préférence par brossage ou friction des cheveux, par exposition à un stimulus, tel que la chaleur, un rayonnement UV, ou par application d'une solution, telle qu'un solvant ou une solution saline.

25. Procédé selon l'une quelconque des revendications 21 à 23 **caractérisé en ce que** la rupture de l'enveloppe des capsules se fait sans action intentionnelle d'un utilisateur, par érosion de l'enveloppe, par rupture sous la contrainte du film formé par la composition, par action de la chaleur ou d'un rayonnement UV.
